(19) **Europäisches Patentamt**
**European Patent Office**
**Office européen des brevets**

(11) **EP 4 307 243 A1**

(12) **EUROPEAN PATENT APPLICATION**

(43) Date of publication:
**17.01.2024 Bulletin 2024/03**

(21) Application number: **22184178.6**

(22) Date of filing: **11.07.2022**

(51) International Patent Classification (IPC):
**G06V 10/44** (2022.01)    **G06V 10/82** (2022.01)

(52) Cooperative Patent Classification (CPC):
**G06V 10/454; G06V 10/82;** G06V 2201/03

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **IB Lab GmbH**
**1140 Wien (AT)**

(72) Inventors:
- **GÖTZ, Christoph**
  **1140 Wien (AT)**
- **TIGRE AVELAR, Maria Carolina**
  **1140 Wien (AT)**
- **BERTALAN, Zsolt**
  **1140 Wien (AT)**

(74) Representative: **SONN Patentanwälte GmbH & Co KG**
**Riemergasse 14**
**1010 Wien (AT)**

(54) **PROCESSING A MEDICAL IMAGE**

(57)    A method for processing a medical image, the method comprising the following steps:
receiving a medical image,
performing an object detection and classification on said medical image,
storing the detected parameters of one or more detected objects in association with the image.

EP 4 307 243 A1

## Description

The present invention concerns a method for processing a medical image, a medical image processing system, a computer program product and a computer-readable medium.

## Problem Statement

Medical images are most commonly exchanged in the Digital Imaging and Communications in Medicine (DICOM) format. This format allows for storing metadata associated with the stored image. However, oftentimes the metadata is incomplete or inaccurate due to lack of appropriate or correct manual data entry. In addition, the DICOM format has limitations as to the structure of the metadata. For example, it does not provide for ambiguous data entry at the cost of compromising completeness.

The problem of incomplete or inconsistent metadata in DICOM images is well-known. The article "Dicom imaging router: An open deep learning framework for classification of body parts from dicom x-ray scans." medRxiv (2021). Pham, Hieu H., Dung V. Do, and Ha Q. Nguyen proposes a DICOM Imaging Router that deploys deep convolutional neural networks (CNNs) for categorizing unknown DICOM X-ray images into five anatomical groups: abdominal, adult chest, pediatric chest, spine, and others. One downside of this approach is that it reliably supports only distinct anatomical groups and only images comprising exactly one of those groups.

## Invention

It is an object of the present invention to support routing information also for medical images containing fully overlapping targets, i.e., where one target is a subset of another. As a downstream effect, supporting fully overlapping targets enables extended and optional embodiments of the present disclosure to include additional information gained through digital image analysis like body implants, metal work (plates, nails, screws) and outside structures (name tags, markers, calibration balls, rulers, ...) into the routing information.

The invention provides a method of the kind defined in the outset, comprising the steps of receiving a medical image, performing an object detection and classification on said medical image, and storing the detected parameters of one or more detected objects in association with the image; thus allowing the detection of multiple objects, and classes of objects, in the same image. More specifically, the method maybe used to store the detected parameters of one or more detected objects into a structured XML file and/or an additional file container in the DICOM format and/or as standard or custom DICOM tags into the input DICOM image. The object detection and classification allows for detecting multiple objects and multiple classes of objects in the same image. This type of metadata cannot usually be stored, or not completely be stored, in the standard DICOM format. However, by using individual private DICOM tags, the obtained information can be sufficiently well mapped. The present disclosure includes writing information, or at least parts of the generated additional information, back into DICOM format files, not being limited to the DICOM format for storage, thus also proposing an extended format, or a related database, comprising associations between medical images (e.g., DICOM images) and further metadata, such as multiple classes of objects and their positions and bounding boxes on the associated images.

The object detection and classification may be configured for detecting and classifying one or more of instances of body parts, instances of body implants and instances of outside structures. The instances of outside structures maybe instances of annotation, measurement and calibration objects. Hence, the method may comprise storing the detected parameters of one or more of instances of body parts, instances of body implants and instances of outside structures in association with the image, for example into a file container in DICOM format. Since body implants (such as a hip implant) naturally often overlap or fully overlap with body parts (such as the bones of the natural hip), it is of particular advantage for the use case, when this information is recognized and can be used in downstream routing of the medical image.

## Configurations

The object classification may be configured to discriminate view position and laterality of the detected objects. The obtained information on view position and laterality may be included in the stored parameters, for example within an XML file or a DICOM file container. This information can be used by viewers and can for instance be displayed by a downstream viewing system. The information on the position laterality may also be used for more specialized routing decisions, for example to processing modules which are specialized on particular configurations of view position and/or laterality.

According to another embodiment of the disclosed method, which can be combined with any of the previous embodiments, the method may further comprise the following steps: providing two or more specialized processing modules, wherein each specialized processing module is associated with one or more compatible mandatory object classes, comparing the one or more detected object classes associated with the image with each of the one or more compatible mandatory object classes to determine at least one matching parameter for each specialized processing module, selecting at least one of the two or more specialized processing modules based on the at least one matching parameter, processing the image with the selected at least one processing module.

## Neural Network

According to an embodiment of the present disclosure, the object detection and classification may use a trained artificial neural network, wherein the training data used for training the artificial neural network comprises medical images with annotated and classified objects, wherein the annotated and classified objects are one or more from a group consisting of body parts, body implants and outside structures. Again, the outside structures may include annotation, measurement and calibration objects.

In this embodiment, the artificial neural network may for example use at least two interdependent convolutional neural networks, wherein a first convolutional neural network is configured and trained for feature extraction and a second convolutional neural network is configured and trained for mapping extracted features to the original image. This allows for different scaling of the medical image and improves recognition of different objects in different parts of the image.

## Logic Module

The disclosed method encompasses a unique logic module responsible for filtering and combining the outputs of the above described network, in order to seamlessly route the input image to a given destination, for example a specialized processing module.

Each destination is characterized by a collection of object classes that can be configured. The logic module compares the detected object classes in the image, with each of the configured destinations, consequently selecting either one or more destinations, or no destination.

The routed image's meta-data is enriched by the findings of the present embodiment. The selected processing module may for example be configured to detect one or more medical conditions and store one or more corresponding labels in association with the image. The stored corresponding labels may be used for display in a viewer of the image, for example in a downstream display and viewing system.

Specialized processing modules may be specialized in particular body parts, such as hips, knees or legs; hence allowing them to be specifically configured to provide support in detecting certain medical conditions related to those particular body parts. A specialized processing module capable of providing such support based on a medical image of a knee, will not provide any meaningful support when served with a medical image of a hip. Therefore, it is desirable to process any medical image only with a suitably specialized processing module. The assignment of a particular medical image to a particular specialized processing module could in principle be performed manually upon viewing the medical image.

In the embodiment mentioned above, the selecting step may use a distance measure applied to the at least one matching parameter and selects exactly one processing module corresponding to the smallest distance measure of the two or more specialized processing modules. The distance measure may take into account multiple matching parameters. It may compare the matching parameters determined for a received medical image with different sets of matching parameters predefined for each of the specialized processing modules. In general, each processing module can be associated with one or multiple predefined sets of matching parameters. If the distance measure is smallest for any of the sets, the associated specialized processing module is being selected.

The distance measure defines a numerical distance between a collection of objects found in the input image, and each collection of objects configured to a destination. The metric may for example be the Hamming distance, which measures the distance of strings, or it can be the number of matching objects. The two most useful distances are:

$$d(x,y) = 0 \text{ if } (x{==}y);$$

$\infty$ else

$$d(x,y) = \dim(\{x_i\} \neg \exists \{y_i\})$$

Each specialized processing module may be associated with zero or more compatible optional object classes, wherein the comparing step may comprise comparing the one or more detected object classes associated with the image with each of the one or more compatible mandatory object classes and each of the zero or more compatible optional object classes to determine the at least one matching parameter for each specialized processing module. For example, each mandatory object class as well as any optional object class may be represented by a separate matching parameter. Together they form a predefined set of matching parameters corresponding to the respective specialized processing module. This predefined set may be compared to a set of matching parameters corresponding to the received medical image as determined during object detection and classification, wherein each detected object class is represented by a separate matching parameter.

According to an extended embodiment, two or more specialized processing modules may be selected based on the at least one matching parameter, wherein the image is processed with all of the selected processing modules, wherein labels corresponding to medical conditions detected by different processing modules are collectively stored in association with the same image or stored in association with separate copies of the image. This provides for the case where the received medical image contains enough information for different specialized processing modules to provide supporting information, usually on different areas are sections of the medical image. In this case it can be useful to process the medical image not only with a single specialized processing module, but with multiple specialized processing modules. Each specialized processing module may receive information obtained through object detection and classification, i.e. the classes and bounding boxes of any detected body parts, body implants or outside structures. The specialized processing module may crop the medical image to a region of interest based on this information. Alternatively, such a cropping may be performed prior to engaging the specialized processing module based on the predefined matching parameters of the respective specialized processing module.

Generally, and with respect to any of the embodiments described above or combinations thereof, the medical image may be a radiographic image, in particular a two-dimensional x-ray image, an ultrasound image, a computer tomography (CT) image, a magnetic resonance (MRT) image, or a positron emission (PET) image. The medical image may be a two-dimensional projection or a two-dimensional slice of a three-dimensional image or model obtained with any of the mentioned imaging techniques.

The medical image may be received in the Digital Imaging and Communications in Medicine (DICOM) format. This format is widespread for medical images and the capability of processing medical images in this format allows to apply and integrate the present method more easily into existing systems for storing, distributing, processing and viewing medical images. One such system is the Picture Archiving and Communication System (PACS).

The present disclosure extends to a medical image processing system comprising means adapted to execute the steps of the method according to any of the embodiments described above or combinations thereof.

The present disclosure extends to a computer program product comprising instructions to cause the system described above to execute the steps of the method according to any of the embodiments described above or combinations thereof.

Finally, the present disclosure extends to a computer-readable medium having stored thereon the computer program described above.

**Example**

In the following, preferred embodiments of the method and the system according to the present disclosure will be discussed for purposes of illustrating the present invention and not for purposes of limiting the same.

According to a first exemplary embodiment, it is proposed a workflow which encompasses getting a DICOM image as input and determining what is represented in it through object detection and classification.

This consists of determining the view position, laterality and body part of the image, other visible objects on the image and corresponding bounding boxes. Afterwards, according to the determined outcomes, a specialized processing module is assigned to the received image.

The object detection and classification determine predefined classes and subclasses as well as and corresponding views, for example:

- Body parts (anatomic structures):

  ○ Hip - Dunn view, anteroposterior (AP)
  ○ Knee - AP/posteroanterior (PA), lateral, skyline, Rosenberg
  ○ Ankle - AP, lateral

- Body implants or metal work:

  ○ Plates
  ○ Nails - tibia, femur
  ○ Screws
  ○ Hip implant
  ○ Knee implant

- Outside structures

  ○ Calibration ball
  ○ Ruler
  ○ Right/Left marker
  ○ Annotation

The specialized processing modules are assigned based on matching parameters determined from their compatible mandatory and optional object classes. The compatible mandatory and optional object classes for two specialized processing modules may be:

- Module H, specialized on hip

  ○ Matching group H1

    ■ Mandatory: Hip left
    ■ Optional: Hip osteotomy left, hip implant left, calibration ball, radiographic protection

  ○ Matching group H2

    ■ Mandatory: Hip right
    ■ Optional: Hip osteotomy right, hip implant right, calibration ball, radiographic protection

  ○ Matching group H3

    ■ Mandatory: Hip left, hip right (i.e., both sides)
    ■ Optional: Hip osteotomy left, hip implant left, hip osteotomy right, hip implant right, calibration ball, radiographic protection

- Module K, specialized on knee

  ○ Matching group K1

    ▪ Mandatory: Knee left
    ▪ Optional: Knee implant left, calibration ball

  ○ Matching group K2

    ▪ Mandatory: Knee right
    ▪ Optional: Knee implant right, calibration ball

  ○ Matching group K3

    ▪ Mandatory: Knee left, knee right
    ▪ Optional: Knee implant left, knee implant right, calibration ball

The used Network in this Example is a slightly-modified version of the one presented by Lin, Tsung-Yi, et al. "Focal loss for dense object detection." Proceedings of the IEEE international conference on computer vision. 2017. This is an object detector network which, for an input image, provides the class, position and confidence scores of the detected objects. These outputs are then logically combined in order to infer the image's content.

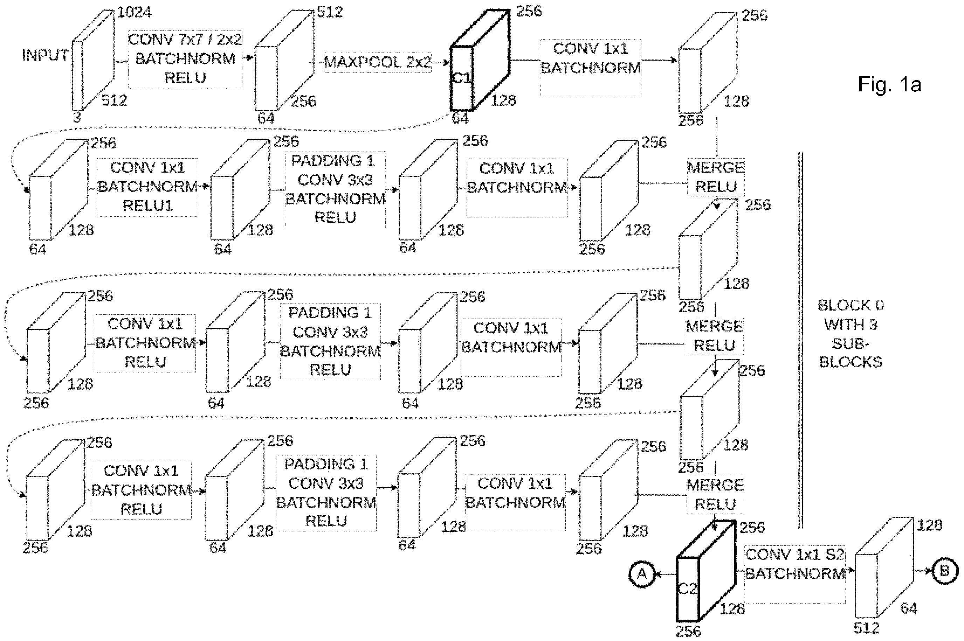

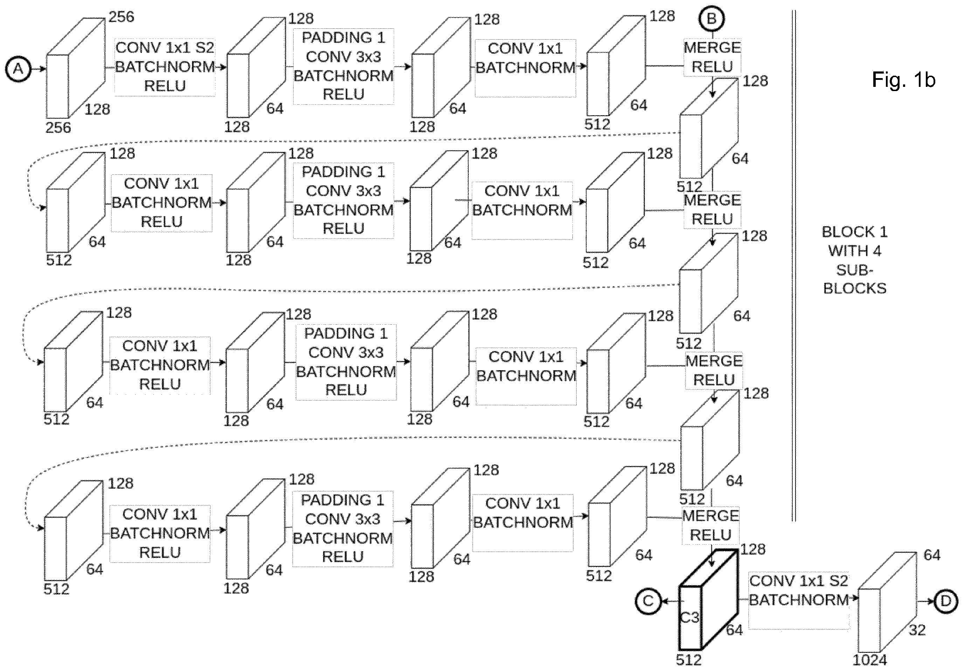

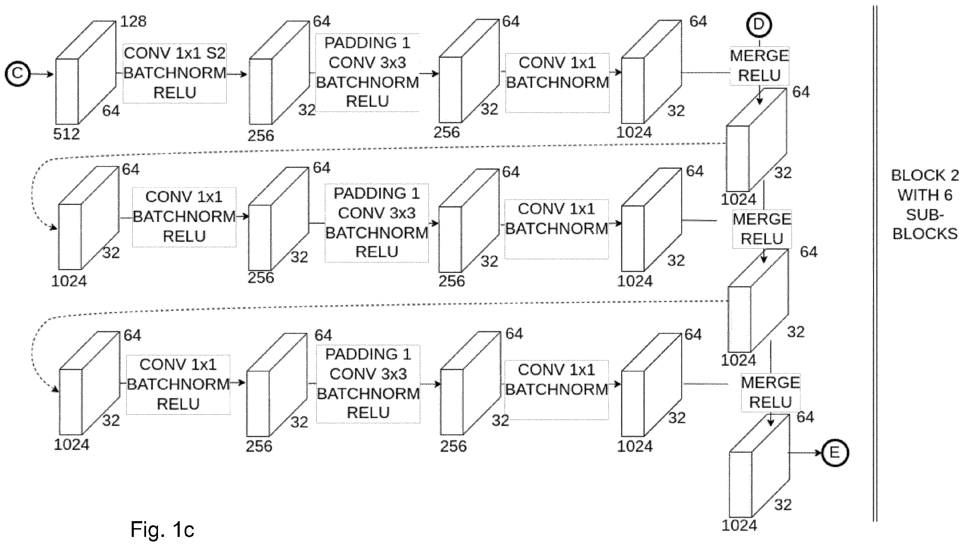

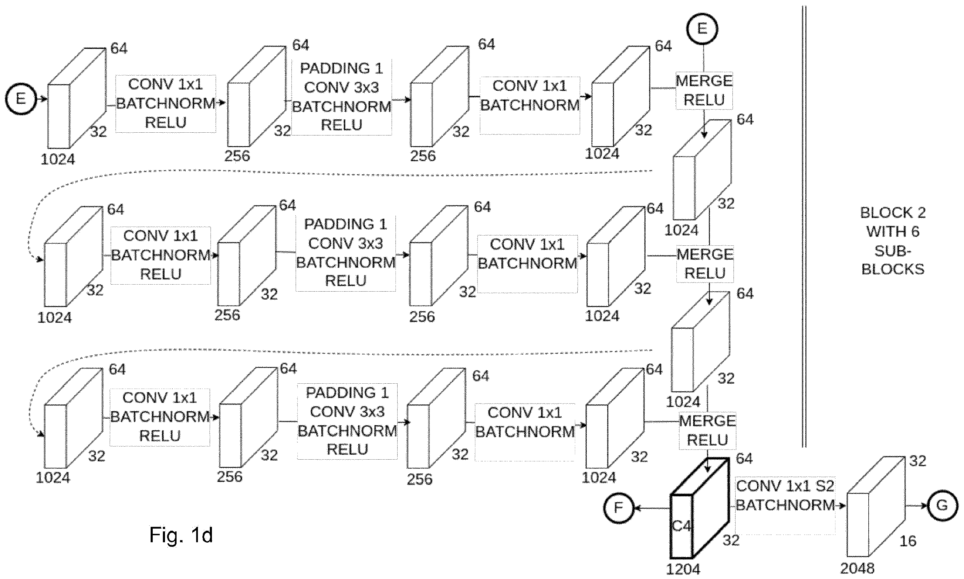

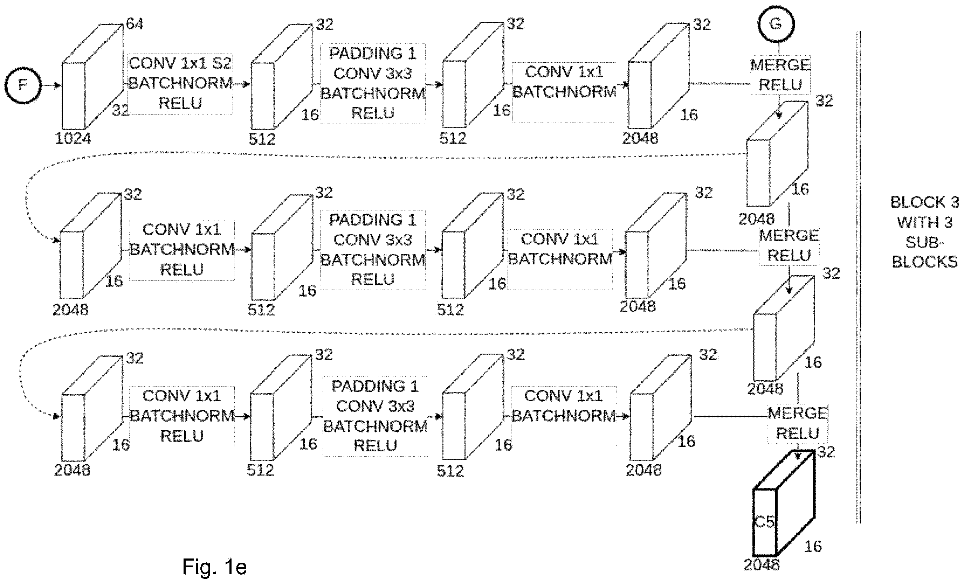

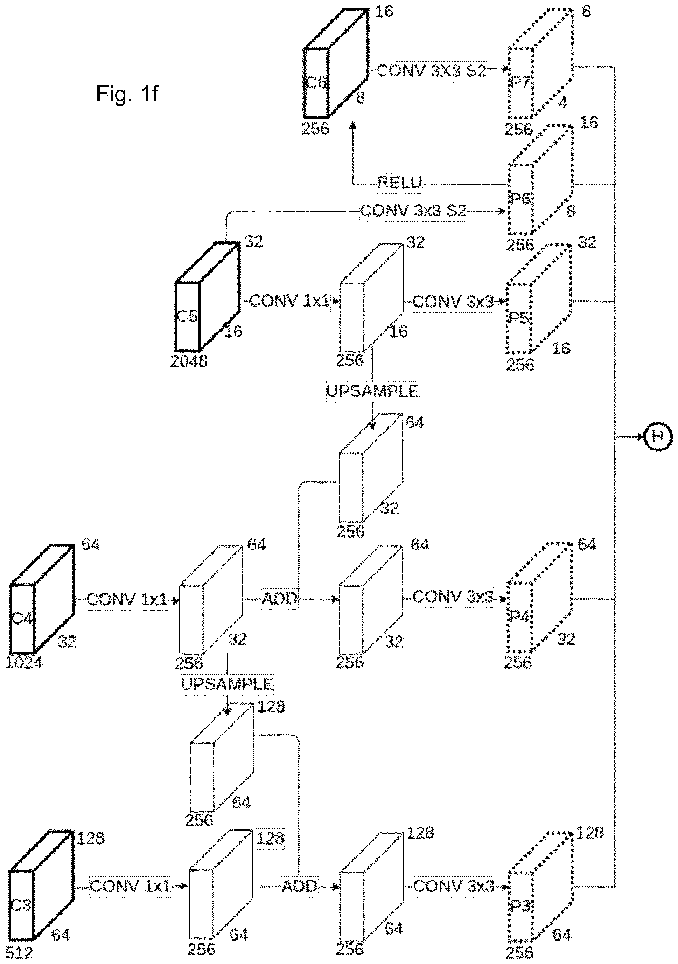

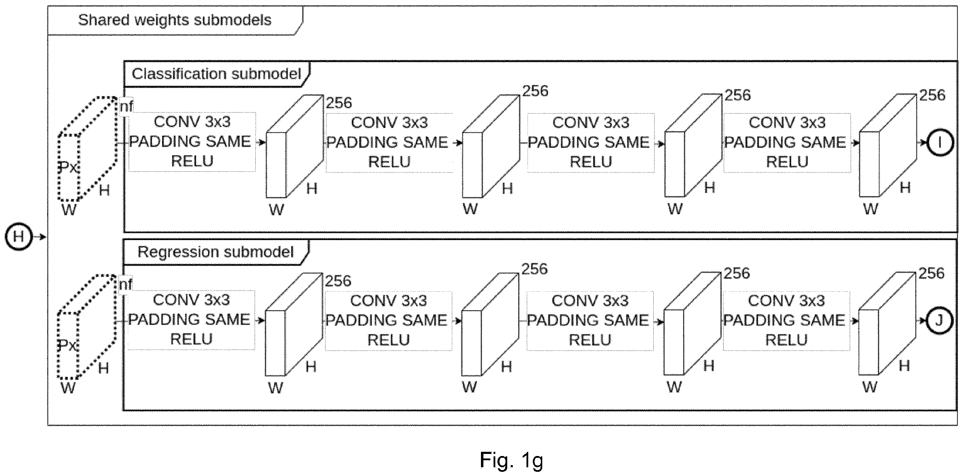

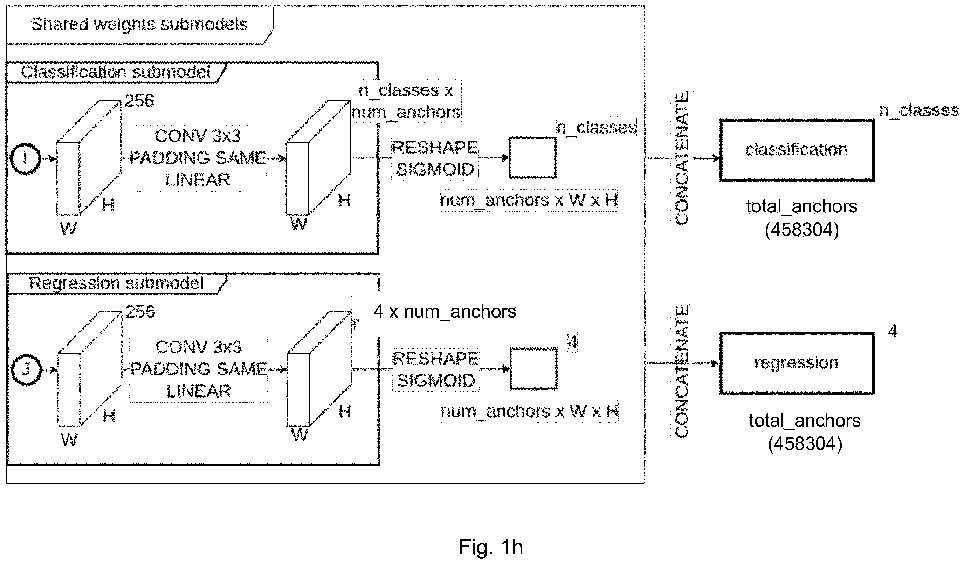

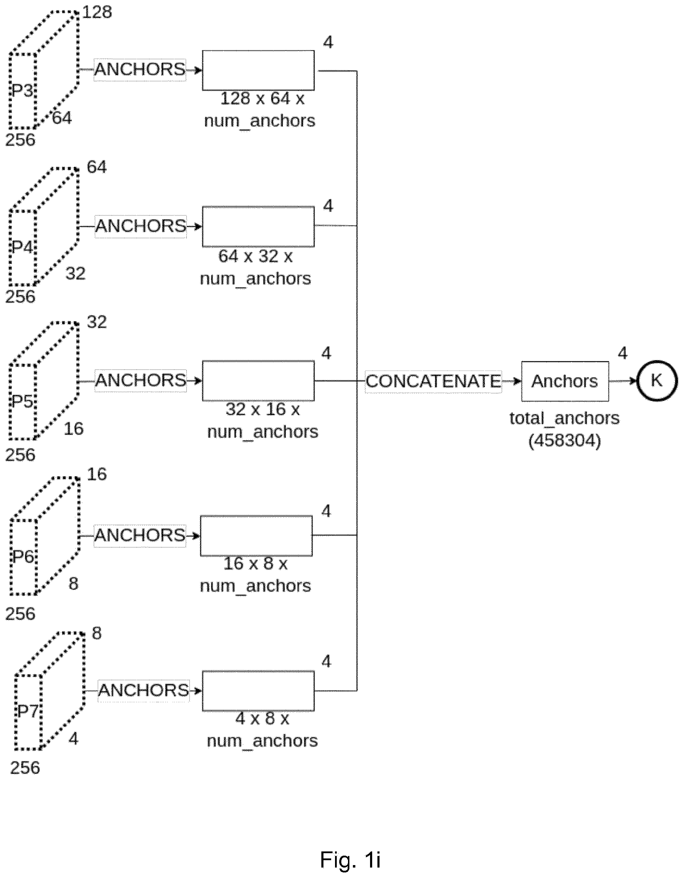

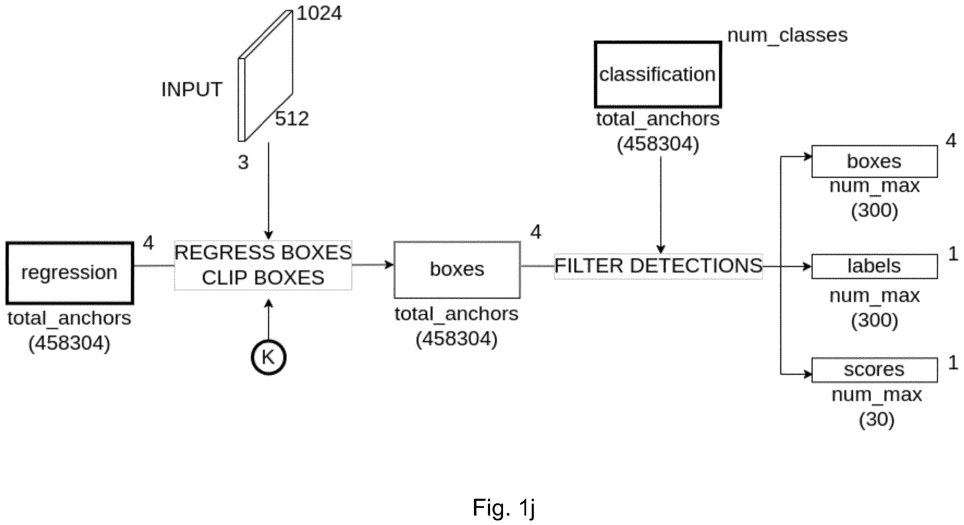

Fig. 1 shows the architecture of the used RetinaNet: a) Architecture of the RetinaNet's Backbone, ResNet50. b) Architecture of the Feature pyramid model (originates the red blocs) and the classification (orange) and regression (purple) submodels. c) Architecture of the pruned model which outputs absolute coordinates for the detected objects.

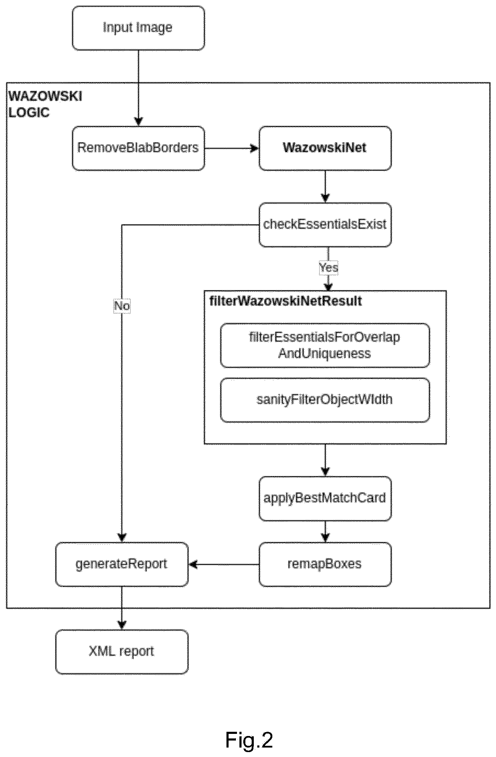

The detailed workflow is schematically shown in Fig. 2 and described below:

1) Crop image to relevant content, apply relevant image pre-processing operations

2) Feed the image to neural network and record result

   a) Each result consists of a collection of found objects. Each object is characterized by: a bounding box, a label and a score. The bounding box is characterized by four floating-point numbers which place it in the image. The label consists of a single integer, representing the object class. Encoded in the image class is: body part, view position and laterality. The score is characterized by a single floating-point number, representing the confidence of the finding.

3) Filter the result according to uniqueness and bounding-box overlap

   a) Body parts can be either unique or not unique. Body parts can be either essential or non-essential.

   b) Find all essential body parts. Remove any duplicate essential body-parts, keeping the one with the highest score.

   c) Find all non-essential body parts. Remove any object whose bounding box overlaps with another instance of this body part. Keep the one with the highest score.

4) Filter the result according to the object widths:

   a) checks if the predicted bounding boxes make sense (e.g., a calibration ball bounding box cannot be bigger than the hip one)

   b) remove objects whose bounding box does not meet these object-size criteria

5) Filter the result according to minimum scores:

   a) remove objects whose scores does not meet the minimum score defined for its label

6) For each thusly filtered result, compare to a pre-defined, configurable dictionary of routing destinations.

   a) The comparison is done via a configurable metric and allows to define a distance between the result and each entry in the dictionary.

   b) The routing destination(s) with the smallest distance(s) is chosen.

   c) It is possible that the metric yields an infinite distance for each routing destination. In that case no routing takes place.

Essential body parts are body parts where at least one is required in the image for the image to make sense and they belong to the group "Body parts (anatomic structures)". Implants, metal work and outside structures are not essential body parts. Classes from those categories can overlap with essential body parts.

**Training Data**

The data used to train the networks in this embodiment has been annotated according to the defined classes consisting of body parts, body implants and outside structures. The current object detection task is done by means of bounding boxes, with an associated class, around the found object. Hereupon, all the classes which are desirable to be detected, refer to the feature description of the specialized processing modules for further details, should be encompassed in the training set.

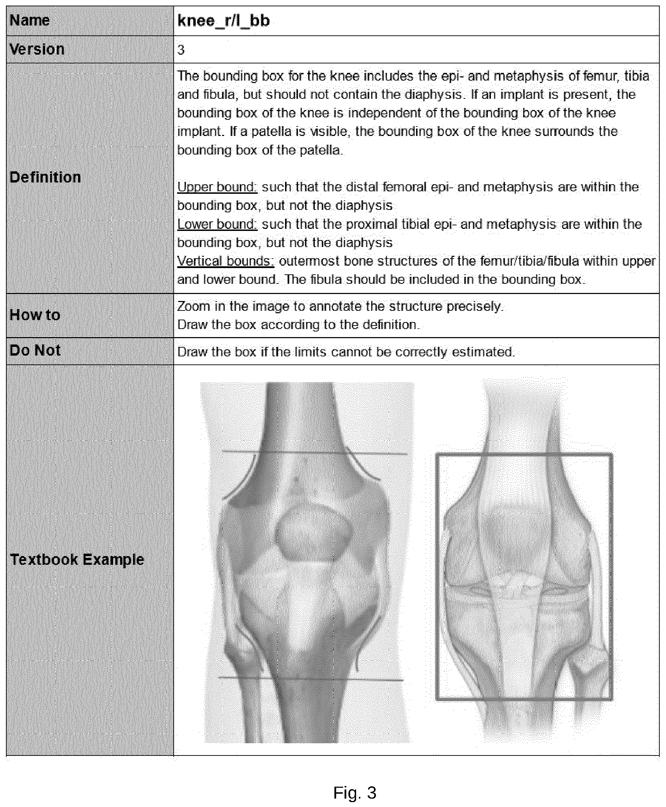

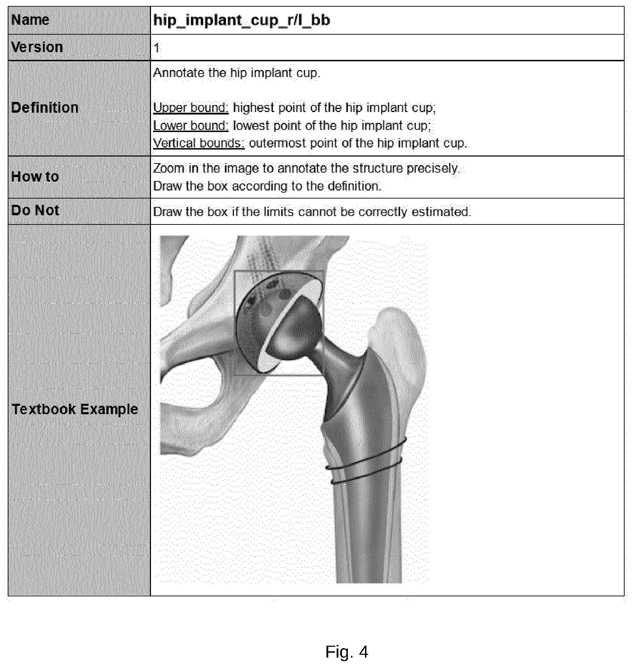

Abounding box is a zero-degree-box which encapsulates a determined object occupying a minimum area for that. It is defined by two points: the top left and the right bottom, both belonging to the box. Figures 3 and 4 showcase examples of annotation requirements and guidelines for two different classes, knee and hip implant cup respectively, encompassed in this task.

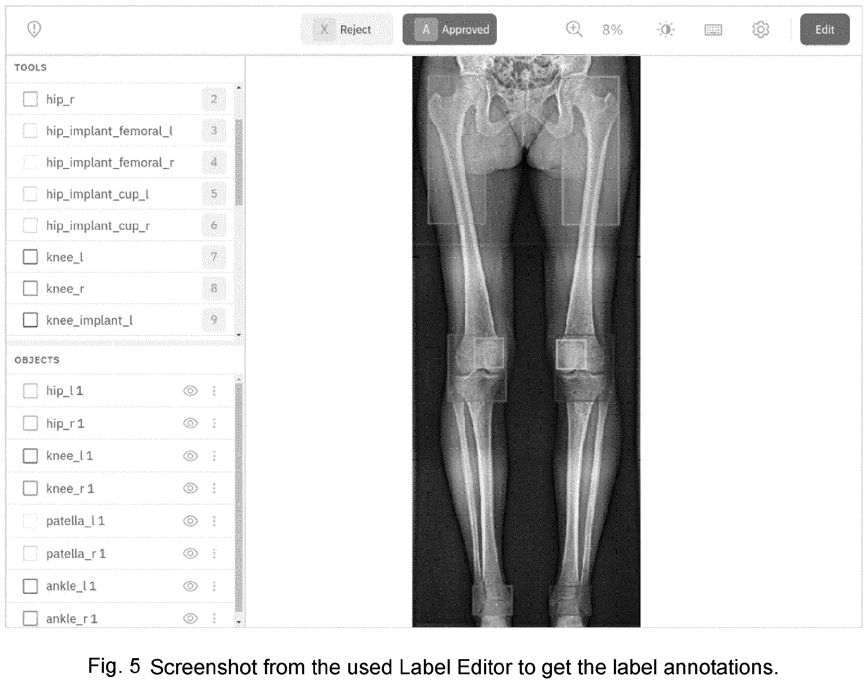

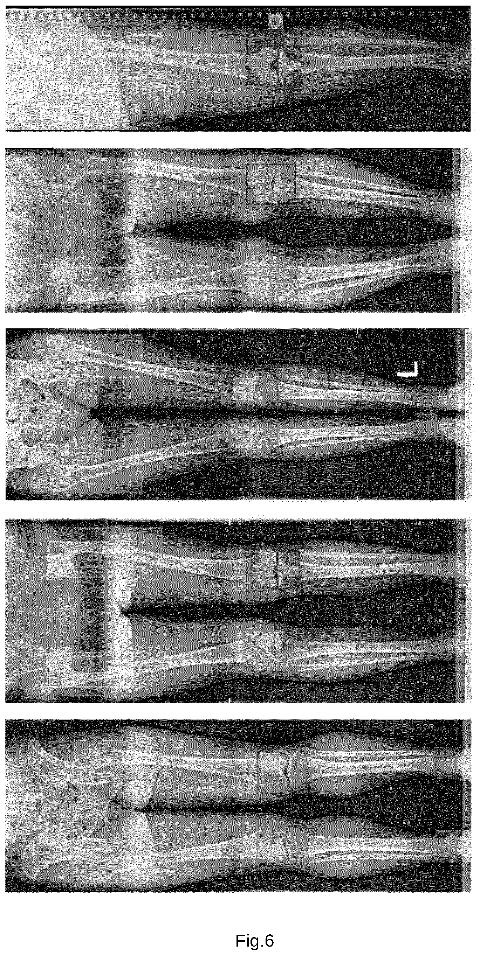

The annotation is performed using a specialized labeling editor. Fig. 5 relates to an exemplary graphical user interface, where the bounding boxes can be drawn with pixel precision. The outcome of such labeling is showcased in Fig.6 where it is visible that the sizes of the bounding boxes are consistent, on a class level, across the different images.

## Claims

1.  A method for processing a medical image, the method comprising the following steps:

    receiving a medical image,
    performing an object detection and classification on said medical image,
    storing the detected parameters of one or more detected objects in association with the image.

2.  The method of claim 1, **characterized in** the object detection and classification is configured for objects detecting and classifying one or more of instances of body parts, instances of body implants and instances of outside structures, in particular annotation, measurement and calibration objects.

3.  The method of claim 2, **characterized in** the object detection and classification uses a trained artificial neural network, wherein the training data used for training the artificial neural network comprises medical images with annotated and classified objects, wherein the annotated and classified objects are one or more from a group consisting of body parts, body implants and outside structures, in particular annotation, measurement and calibration objects.

4.  The method of claim 3, **characterized in that** the artificial neural network uses at least two interdependent convolutional neural networks, wherein a first convolutional neural network is configured and trained for feature extraction and a second convolutional neural network is configured and trained for mapping extracted features to the original image.

5.  The method of any one of claims 2 to 4, **characterized in that** the object classification is configured to discriminate view position and/or laterality of the detected objects.

6.  The method of any one of claims 1 to 5, further comprising the following steps:

    providing two or more specialized processing modules, wherein each specialized processing module is associated with one or more compatible mandatory object classes;
    comparing the one or more detected object classes associated with the image with each of the one or more compatible mandatory object classes to determine at least one matching parameter for each specialized processing module,
    selecting at least one of the two or more specialized processing modules based on the at least one matching parameter,
    processing the image with the selected at least one processing module, wherein the selected processing module detects one or more medical conditions and stores one or more corresponding labels in association with the image for displaying to a viewer of the image.

7.  The method of claim 6, **characterized in that** the selecting step uses a distance measure applied to the at least one matching parameter and selects exactly one processing module corresponding to the smallest distance measure of the two or more specialized processing modules.

8.  The method of claim 6 or 7, **characterized in that** each specialized processing module is associated with zero or more compatible optional object classes, wherein the comparing step comprises comparing the one or more detected object classes associated with the image with each of the one or more compatible mandatory object classes and each of the zero or more compatible optional object classes to determine the at least one matching parameter for each specialized processing module.

9.  The method of any one of claims 6 to 8, **characterized in that** two or more specialized processing modules are selected based on the at least one matching parameter, wherein the image is processed with all of the selected processing modules, wherein labels corresponding to medical conditions detected by different processing modules are collectively stored in association with the same image or stored in association with separate copies of the image.

10. The method of any one of claims 1 to 9, **characterized in that** the medical image is a radiographic image, in particular a two-dimensional x-ray image, an ultrasound image, a computer tomography image, a magnetic resonance image, or a positron emission image.

11. The method of any one of claims 1 to 10, **characterized in that** the medical image is received in the Digital Imaging and Communications in Medicine (DICOM) format.

12. A medical image processing system comprising means adapted to execute the steps of the method of any one of claims 1 to 11.

13. A computer program product comprising instructions to cause the system of claim 11 to execute the steps of the method of any one of claims 1 to 11.

14. A computer-readable medium having stored thereon the computer program of claim 13.

Fig. 1a

Fig. 1b

Fig. 1c

BLOCK 2 WITH 6 SUB-BLOCKS

Fig. 1d

Fig. 1e

BLOCK 3 WITH 3 SUB-BLOCKS

Fig. 1f

Fig. 1g

Fig. 1h

EP 4 307 243 A1

Fig. 1i

Fig. 1j

EP 4 307 243 A1

Fig.2

| Name | knee_r/l_bb |
|---|---|
| Version | 3 |
| Definition | The bounding box for the knee includes the epi- and metaphysis of femur, tibia and fibula, but should not contain the diaphysis. If an implant is present, the bounding box of the knee is independent of the bounding box of the knee implant. If a patella is visible, the bounding box of the knee surrounds the bounding box of the patella.<br><br>Upper bound: such that the distal femoral epi- and metaphysis are within the bounding box, but not the diaphysis<br>Lower bound: such that the proximal tibial epi- and metaphysis are within the bounding box, but not the diaphysis<br>Vertical bounds: outermost bone structures of the femur/tibia/fibula within upper and lower bound. The fibula should be included in the bounding box. |
| How to | Zoom in the image to annotate the structure precisely.<br>Draw the box according to the definition. |
| Do Not | Draw the box if the limits cannot be correctly estimated. |
| Textbook Example | |

Fig. 3

| Name | hip_implant_cup_r/l_bb |
|---|---|
| Version | 1 |
| Definition | Annotate the hip implant cup.<br><br>Upper bound: highest point of the hip implant cup;<br>Lower bound: lowest point of the hip implant cup;<br>Vertical bounds: outermost point of the hip implant cup. |
| How to | Zoom in the image to annotate the structure precisely.<br>Draw the box according to the definition. |
| Do Not | Draw the box if the limits cannot be correctly estimated. |
| Textbook Example | |

Fig. 4

Fig. 5 Screenshot from the used Label Editor to get the label annotations.

Fig.6

Europäisches
Patentamt

European
Patent Office

Office européen
des brevets

## EUROPEAN SEARCH REPORT

**Application Number**

EP 22 18 4178

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/166807 A1 (QUENNESSON KEVIN [US] ET AL) 3 June 2021 (2021-06-03) | 1-4,6-14 | INV. G06V10/44 |
| Y | * paragraph [0044] - paragraph [0045] * * paragraph [0164] - paragraph [0168] * * paragraph [0173] * * paragraph [0175] * * paragraph [0186] * * paragraph [0189] * | 5 | G06V10/82 |
| Y | US 2022/101984 A1 (PARK JONG CHAN [KR] ET AL) 31 March 2022 (2022-03-31) * paragraph [0066] * * paragraph [0103] * * paragraph [0107] * * paragraph [0146] - paragraph [0147] * | 5 | |
| A | US 2020/352518 A1 (LYMAN KEVIN [US] ET AL) 12 November 2020 (2020-11-12) * paragraph [0100] * * paragraph [0243] * * paragraph [0263] * * paragraph [0266] - paragraph [0267] * * paragraph [0270] * * paragraph [0288] * * paragraph [0291] * * paragraph [0297] * | 1-14 | |

**TECHNICAL FIELDS SEARCHED (IPC)**

G06V

The present search report has been drawn up for all claims

| Place of search | Date of completion of the search | Examiner |
|---|---|---|
| The Hague | 28 November 2022 | Koutroumpas, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 18 4178**

This annex lists the patent family members relating to the patent documents cited in the above-mentioned European search report.
The members are as contained in the European Patent Office EDP file on
The European Patent Office is in no way liable for these particulars which are merely given for the purpose of information.

**28-11-2022**

| Patent document cited in search report | | Publication date | Patent family member(s) | | Publication date |
|---|---|---|---|---|---|
| US 2021166807 | A1 | 03-06-2021 | US | 2021166466 A1 | 03-06-2021 |
| | | | US | 2021166807 A1 | 03-06-2021 |
| | | | WO | 2021108771 A1 | 03-06-2021 |
| US 2022101984 | A1 | 31-03-2022 | CN | 111986784 A | 24-11-2020 |
| | | | EP | 3742451 A1 | 25-11-2020 |
| | | | EP | 3975197 A1 | 30-03-2022 |
| | | | JP | 6895508 B2 | 30-06-2021 |
| | | | JP | 2020191063 A | 26-11-2020 |
| | | | KR | 102075293 B1 | 07-02-2020 |
| | | | US | 10824908 B1 | 03-11-2020 |
| | | | US | 2021012160 A1 | 14-01-2021 |
| | | | US | 2022101984 A1 | 31-03-2022 |
| | | | US | 2022269905 A1 | 25-08-2022 |
| | | | WO | 2020235966 A1 | 26-11-2020 |
| US 2020352518 | A1 | 12-11-2020 | NONE | | |

EPO FORM P0459

For more details about this annex : see Official Journal of the European Patent Office, No. 12/82